# EUROPÄISCHE PATENTANMELDUNG

(11) **EP 2 187 196 A2**
(43) Veröffentlichungstag der Anmeldung: **19.05.2010**
(21) Anmeldenummer: 09005339.8
(22) Anmeldetag: 14.04.2009
(51) Int. Cl.: G01N 3/60, G01N 33/20

(54) **Vorrichtung zur Bestimmung der Heißrissempfindlichkeit von metallischen Schmelzen**

(30) Priorität: 04.07.2008 DE 102008031777
(71) Anmelder: GKSS-Forschungszentrum Geesthacht GmbH, 21502 Geesthacht (DE)
(72) Erfinder: Zhen, Zisheng, 21502 Geesthacht (DE); Utke, Oliver, 23795 Bad Segeberg (DE); Punessen, Willi, 21502 Geesthacht (DE); Hort, Norbert, 21339 Lüneburg (DE); Kainer, Karl, Ulrich, Prof. Dr., 21522 Hohnstorf (DE)
(74) Vertreter: UEXKÜLL & STOLBERG

(57) **Zusammenfassung**

Die vorliegende Erfindung betrifft eine Vorrichtung zur Bestimmung der Heißrissempfindlichkeit von metallischen Schmelzen mit einer Gießform, die ein erstes Teilvolumen und ein zweites Teilvolumen aufweist, wobei das zweite Teilvolumen als ein rohrförmiger Abschnitt mit einem ersten Ende und einem zweiten Ende ausgebildet ist, wobei das erste Ende mit dem ersten Teilvolumen verbunden ist, mit einem Messelement, das sich in das zweite Ende des rohrförmigen Abschnitts erstreckt und mit einer Messeinrichtung zum Erfassen einer Kraft auf das Messelement und/oder einer Positionsänderung des Messelements in Erstreckungsrichtung des rohrförmigen Abschnitts, wobei die Messeinrichtung mit dem Messelement gekoppelt ist. Die Aufgabe, eine verbesserte quantitative und qualitative Ermittlung der Heißrissempfindlichkeit zu erlauben, wird dadurch gelöst, dass sich die Querschnittsfläche des rohrförmigen Abschnitts in jedem im Wesentlichen über die gesamte Länge beliebig auswählbaren Teilabschnitt des rohrförmigen Abschnitts in Richtung des zweiten Endes hin verringert.

## Beschreibung

Die vorliegende Erfindung betrifft eine Vorrichtung zur Bestimmung der Heißrissempfindlichkeit von metallischen Schmelzen mit einer Gießform, die ein erstes Teilvolumen und ein zweites Teilvolumen aufweist, wobei das zweite Teilvolumen als ein rohrförmiger Abschnitt mit einem ersten Ende und einem zweiten Ende ausgebildet ist, wobei das erste Ende mit dem ersten Teilvolumen verbunden ist, mit einem Messelement, das sich in das zweite Ende des rohrförmigen Abschnitts erstreckt und mit einer Messeinrichtung zum Erfassen einer Kraft auf das Messelement und/oder einer Positionsänderung des Messelements in Erstreckungsrichtung des rohrförmigen Abschnitts, wobei die Messeinrichtung mit dem Messelementgekoppelt ist.

Heißrisse sind ein bedeutsames Problem bei der Erstarrung metallischer Schmelzen. Als Heißrisse werden interkristallin oder interdendritisch verlaufende Werkstofftrennungen bezeichnet, die beim Erstarren oder Aufschmelzen in einem Temperaturbereich zwischen Solidus- und Liquidustemperatur entstehen. Heißrisse können mikroskopisch klein sein oder sich über mehrere Millimeter oder gar Zentimeter erstrecken. In jedem Fall stellen sie eine Schadstelle für ein Werkstück dar, das zumindest teilweise aus einer erstarrten metallischen Schmelze entstehen soll.

Besonders im Hinblick auf Bandguss- oder vergleichbare kontinuierliche Gießverfahren ist die Kenntnis über die Empfindlichkeit einer Schmelze, Heißrisse auszubilden, von großer Bedeutung, da dies einen unmittelbaren Einfluss auf die Festigkeit des gegossenen Produkts hat. Auch für Schweißnähte bzw. die Verwendung von bestimmten Schweißverfahren ist diese Kenntnis von Bedeutung, da dies einen unmittelbaren Einfluss auf die Haltbarkeit der Schweißnaht hat.

In der Vergangenheit hat es vor allem bei Stählen und Aluminiumlegierungen erhebliche Aktivitäten zur Bestimmung der Heißrissempfindlichkeit gegeben. Bislang ist es jedoch noch nicht gelungen, das Phänomen der Heißrissempfindlichkeit hinreichend zu verstehen oder gar zu simulieren. Außerdem fehlt es an einer Vorrichtung, mit der die Heißrissempfindlichkeit zuverlässig bestimmt werden kann.

Typischerweise werden bisher bei einem Erstarrungsprozess einer metallischen Schmelze in einer Gießform neben der chemischen Zusammensetzung auch Schmelzparameter wie die Schmelzbadtemperatur, die Gießformtemperatur, der Verlauf der Temperatur bei der Erstarrung und die bei der Erstarrung in der Gießform durch Schrumpfung entstehenden Kräfte gemessen und bei der Auswertung berücksichtigt. Es kann auch eine rein qualitative Bewertung durch optische Betrachtung des Werkstoffes im Hinblick auf das Auftreten eines Risses, dessen Größe und Morphologie erfolgen.

Ein Heißriss wird hinsichtlich seiner Größe qualitativ charakterisiert. Man unterscheidet dabei in der Regel Risse, die mit dem bloßen Auge zu erkennen sind, mit einem Hilfsmittel wie z.B. einer Lupe sichtbar gemacht werden können oder die nur unter dem Mikroskop erkennbar sind. Auch wird sehr häufig die Risslänge als Kriterium für die Heißrissempfindlichkeit herangezogen. Den bekannten Prüfmethoden ist gemeinsam, dass sie zwar das Auftreten von Rissen und ihre Größe beurteilen können, nicht jedoch Rückschlüsse auf Dehnungen und damit auf resultierende Spannungen zulassen. Durch die Kenntnis von Dehnungen und Spannungen lässt sich allerdings die Empfindlichkeit für noch zukünftig auftretende Risse quantitativ bestimmen oder deren weiteres Verhalten vorhersagen.

Die Heißrissempfindlichkeit wird bislang quantitativ durch Gießformen ermittelt, in denen zum Beispiel eine Fließlänge gemessen wird. Die Länge des erstarrten Materials bis zu der noch kein Riss auftritt, dient dabei als Kennziffer. Dieser Zahlenwert ist spezifisch für die Geometrie der Gießform, d.h. dem Querschnitt des Gießkanals und dem gewählten Angusssystem. Spezifisch für den jeweiligen Versuchsaufbau ist auch das Material, aus dem die Gießform gefertigt ist. Dies kann sowohl z.B. Stahl sein, wenn die Gießform z.B. als Dauerform ausgelegt ist, oder auch Sand. Im letzteren Fall sind als weitere Parameter noch die Korngröße des Sandes, die Korngrößenverteilung, Feuchtigkeit, Bindemittel, Verdichtung und Zusammensetzung des Sandes zu berücksichtigen. Als zusätzliche Kennwerte werden noch die Schmelzbadtemperatur und die Gießformtemperatur ermittelt.

Eine bekannte Gießform zur Bestimmung der Heißrissempfindlichkeit baut auf ringförmigen Geometrien auf, bei denen der Außendurchmesser konstant gehalten wird, während der Innendurchmesser variiert werden kann. Bei der Erstarrung schrumpft das Material auf den Kern zusammen und in Abhängigkeit von Wandstärke, Schmelzbadtemperatur, Gießformtemperatur und chemischer Zusammensetzung können sich Risse bilden. Probleme bei dieser Art der Gießform ergeben sich vor allem im Bereich des Angusses, da die Bedingungen hier häufig nicht reproduzierbar sind.

In S. Instone et al., "New apparatus for characterising tensile strength development and hot cracking in the mushy zone", International Journal of Cast Metals Research 12 (2000) 441-456, ist eine H-formige Gießform mit dem Anguss im Zentrum beschrieben, bei der eine Zugprüfmaschine mit in das System eingebunden wird. Über sie wird eine Kraft eingebunden und unter Berücksichtigung des bei einer bestimmten Kraft auftretenden Risses und bekannten Temperaturprofilen wird versucht, Rückschlüsse auf die Heißrissempfindlichkeit zu ziehen.

Daneben sind auch Gießformen mit einem ersten Aufnahmevolumen und einem rohrförmigen Messvolumen im Einsatz. Dabei treten im Verbindungsbereich zwischen dem ersten Aufnahmevolumen und dem rohrförmigen Messvolumen zumeist Hotspots auf. Ein Hotspot ist eine Anhäufung von Schmelze während des Erstarrungprozesses, der zumeist in einem zentralen Volumenbereich der Gießform auftritt, der den weitesten Abstand zur kälteren Gießformwandung hat. Bei diesen Gießformen wird das Schrumpfungsverhalten der Schmelze während des Erstarrens über die Länge des Stabes gemessen. Parameter sind auch hier Schmelzbad- und Gießformtemperatur und chemische Zusammensetzung der Schmelze. Durch den Wärmeinhalt von Hotspots wird die Heißrissempfindlichkeit zusätzlich beeinflusst.

Eine Vorrichtung gemäß dem Oberbegriff des Anspruchs ist in G. Cao, S. Kou, "Hot tearing of ternary Mg-Al-Ca alloy castings", Metallurgical and Materials Transactions 37A (2006) 3647-3663, beschrieben. Cao und Kou beschreiben darin eine direkte Messung der bei der Erstarrung auftretenden Dehnungen und der daraus resultierenden Kräfte mit einem stabförmigen Teilvolumen einer Gießform. Auch Y. Wang et al., "An understanding of the hot tearing mechanism in AZ91 magnesium alloy", Materials Letters 53 (2002) 35-39, beschreiben einen Testaufbau mit einer stabförmigen Gießformgeometrie zur Bestimmung der Heißrissempfindlichkeit.

Bei den verwendeten Vorrichtungen kommt es allerdings zu erheblichen Reibungskräften zwischen dem erstarrenden Material und der Innenwand der Gießform, die von den Temperaturen des erstarrenden Materials und der Gießform und letztlich vom Schrumpfungsverhalten beider abhängt. Unter Umständen eingesetzte Schmiermittel bringen weitere Schwierigkeiten bei der Deutung der Messwerte mit sich. Eine sinnvolle Interpretation ist bei derart auftretenden Reibungskräften kaum möglich.

Alle bisher bekannten Vorrichtungen lassen zwar in gewisser Weise eine Beurteilung der Heißrissempfindlichkeit zu. Keine ist jedoch wegen der auftretenden Reibungskräfte zur genauen quantitativen und qualitativen Ermittlung der Heißrissempfindlichkeit geeignet.

Daher ist es die Aufgabe der vorliegenden Erfindung, eine Vorrichtung zur Bestimmung der Heißrissempfindlichkeit von metallischen Schmelzen bereitzustellen, die eine verbesserte quantitative und qualitative Ermittlung der Heißrissempfindlichkeit von verschiedenen metallischen Schmelzen erlaubt.

Diese Aufgabe wird durch eine Vorrichtung gemäß den Merkmalen des Anspruchs 1 gelöst. Vorteilhafte Ausführungsformen sind Gegenstand der abhängigen Unteransprüche.

Gemäß der vorliegenden Erfindung wird eine Vorrichtung zur Bestimmung der Heißrissempfindlichkeit von metallischen Schmelzen mit einer Gießform bereitgestellt, die ein erstes Teilvolumen und ein zweites Teilvolumen aufweist, wobei das zweite Teilvolumen als ein rohrförmiger Abschnitt mit einem ersten Ende und einem zweiten Ende ausgebildet ist, wobei das erste Ende mit dem ersten Teilvolumen verbunden ist, mit einem Messelement, das sich in das zweite Ende des rohrförmigen Abschnitts erstreckt und mit einer Messeinrichtung zum Erfassen einer Kraft auf das Messelement und/oder einer Positionsänderung des Messelements in Erstreckungsrichtung des rohrförmigen Abschnitts, wobei die Messeinrichtung mit dem Messelement gekoppelt ist, dadurch gekennzeichnet, dass sich die Querschnittsfläche des rohrförmigen Abschnitts in jedem im Wesentlichen über die gesamte Länge beliebig auswählbaren Teilabschnitt des rohrförmigen Abschnitts in Richtung des zweiten Endes hin verringert.

Der Begriff "Gießform" umfasst hierbei sämtliche Behältnisse, die dazu geeignet sind, eine metallische Schmelze in einem Innenvolumen zu enthalten, zu empfangen oder darin zu erzeugen.

Der Begriff "Volumen" bedeutet hier im Wesentlichen das Innenvolumen, das für die Aufnahme von Schmelze zur Verfügung steht. Das Innenvolumen der Gießform setzt sich hierbei aus mindestens zwei Teilvolumina zusammen.

Das kennzeichnende Merkmal, dass sich die Querschnittsfläche des rohrförmigen Abschnitts in jedem im Wesentlichen über die gesamte Länge beliebig auswählbaren Teilabschnitt des rohrförmigen Abschnitts in Richtung des zweiten Endes hin verringert, ist im Wesentlichen so zu interpretieren, dass es im Wesentlichen über die gesamte Länge des rohrförmigen Abschnitts keinen Teilabschnitt gibt, bei dem die Querschnittsfläche in Erstrekkungsrichtung des rohrförmigen Abschnitts konstant ist. Damit wird vermieden, dass Reibungskräfte zwischen dem erstarrenden Material und der Gießformwandung die Messung verfälschen. Beispielsweise kann der rohrförmige Abschnitt einen kreisförmigen Querschnitt aufweisen, wobei sich der Durchmesser in jedem Teilabschnitt zum zweiten Ende hin verringert, der Abschnitt also kegelstumpfförmig ist.

Gegenüber bekannten Messvorrichtungen hat die erfindungsgemäße Vorrichtung vor allem den Vorteil, dass das Schrumpfen des erstarrenden Materials relativ zur Gießform, das sich vornehmlich in Erstreckungsrichtung des rohrförmigen Abschnitts auswirkt, dazu führt, dass sich das erstarrende Material sofort von der Gießformwandung löst und somit keine Reibungseffekte zwischen der erstarrenden Schmelze und der Gießformwandung gemessen werden.

Außerdem ist die Messung unabhängig vom Schrumpfungsverhalten der Gießform selbst, da in der Regel die Schmelze in der Gießform stärker schrumpft als das Material der Gießform selbst.

Die erfindungsgemäße Vorrichtung wurde zwar in erster Linie für die Ermittlung der Heißrissempfindlichkeit von Magnesiumschmelzen entwickelt. Bedingt durch den Versuchsaufbau und die ermittelten Kennwerte kann die Vorrichtung allerdings nicht nur für Magnesiumlegierungen, sondern auch für Aluminium-, Zink-, Buntmetalle oder auch für Stähle und andere metallische Schmelzen eingesetzt werden.

Durch die erfindungsgemäße Geometrie und die Möglichkeit, unterschiedlichste Gießformwerkstoffe zu verwenden, lässt die Vorrichtung im Wesentlichen Messungen an allen Werkstoffen während der Erstarrung zu. Damit steht erstmalig eine normierbare Messzelle zur Ermittlung der Heißrissempfindlichkeit zur Verfügung. Es ist daher nicht nur eine qualitative sondern auch gleichzeitig eine quantitative Beurteilung möglich. Dies erlaubt erstmalig auch den direkten Vergleich der Heißrissempfindlichkeit unterschiedlichster Werkstoffe.

Ein großer Vorteil der Erfindung liegt in der gleichzeitigen Detektion von Rissen und den daraus resultierenden Dehnungs- bzw. Spannungsänderungen in einem Probekörper. Es lassen sich sowohl quantitative als auch qualitative Aussagen hinsichtlich der Heißrissempfindlichkeit treffen.

Neben der chemischen Zusammensetzung werden mit der Vorrichtung auch Schmelzparameter wie Schmelzbadtemperatur, Gießformtemperatur, Verlauf der Temperatur bei der Erstarrung und die bei der Erstarrung in der Gießform durch Schrumpfung entstehenden Kräfte gemessen und bei der Auswertung berücksichtigt. Daneben kann gleichzeitig eine rein qualitative Bewertung des Werkstoffes im Hinblick auf das Auftreten eines Risses, dessen Größe, Morphologie und zeitlichem Verhalten erfolgen.

Um hydrostatische Druckeinflüsse oder sonstige durch die Gravitationskraft bedingte Beeinflussungen der Messung zu minimieren, ist es vorteilhaft, wenn der rohrförmige Abschnitt im Wesentlichen in horizontaler Richtung verläuft.

Vorzugsweise sind entlang der Erstreckungsrichtung des rohrförmigen Abschnitts Temperaturmesselemente angeordnet. Diese können zum einen dazu dienen, die Temperaturen zu Auswertungszwecken aufzuzeichnen. Zum anderen können sie dazu verwendet werden, die Temperatur der Gießformwandung und entsprechend die Temperatur der erstarrenden Schmelze zu regeln. Dazu ist es von Vorteil, wenn zumindest ein Temperaturmesselement in dem ersten Teilvolumen angeordnet ist. Dieses kann beispielsweise teleskopisch in das Innenvolumen der Gießform einführbar sein, damit man die Temperatur am Hotspot im Verbindungsbereich zwischen dem ersten und dem zweiten Teilvolumen messen kann.

Für die Temperaturregelung und -steuerung ist es vorteilhaft, wenn das erste Teilvolumen und/oder der rohrförmige Abschnitt mit einer Heizvorrichtung und/oder einer Kühlvorrichtung versehen ist. Dadurch kann der Erstarrungsprozess der Schmelze gezielt gesteuert werden. Es ist dabei besonders vorteilhaft, wenn der rohrförmige Abschnitt so temperiert werden kann, dass das zweite Ende kälter als das erste Ende ist und somit die Schmelze zunächst am zweiten Ende erstarrt und sich dabei zugfest mit dem Messelement verbindet. Dadurch kann die Messung der Zugkraft, die durch das Schrumpfungsverhalten der Schmelze entsteht, von einem möglichst frühen Stadium des Erstarrungsprozesses an genau gemessen werden.

Vorzugsweise ist am zweiten Ende des rohrförmigen Abschnitts eine Lüftungsöffnung vorgesehen, damit die Luft im vorteilhafterweise im Wesentlichen in horizontaler Richtung verlaufenden rohrförmigen Abschnitt entweichen kann, wenn die Schmelze in den Abschnitt eintritt. Diese Lüftungsöffnung kann beispielsweise eine in Erstreckungsrichtung des Messelements oberseitig verlaufende Kerbe im Messelement sein.

Vorzugsweise weist die Vorrichtung ein zumindest teilweise oberhalb der Gießform befindliches Angusssystem auf, wobei das erste Teilvolumen der Gießform dazu ausgestaltet ist, eine metallische Schmelze vom Angusssystem aufzunehmen. Dabei wird in dem Angusssystem die Schmelze erzeugt und bei geeigneter Temperatur der Schmelze und der Gießform die Schmelze durch einen unteren Auslass in das ertse Teilvolumen der Gießform abgelassen. Daher weist vorteilhafterweise das erste Teilvolumen eine obere Öffnung auf zur Aufnahme der Schmelze aus dem darüber befindlichen Angusssystem. Sobald der Schmelzpegel den Verbindungsbereich zwischen ersten und zweitem Teilvolumen der Gießform erreicht hat, verteilt sich die Schmelze auch in das zweite Teilvolumen.

Damit sich das Messelement möglichst reibungsfrei in axialer Richtung bewegen kann, ist es relativ zur Gießform vorzugsweise in einer Hülse, beispielsweise aus Graphit oder Keramik, mit geringem Reibungskontakt geführt.

Für die Auslese und Auswertung der Messergebnisse sind die Messeinrichtung und ggf. die Temperaturmesselemente vorzugsweise mit einem computergesteuerten Auslesesystem verbunden und mit diesem auslesbar.

Im Folgenden werden vorteilhafte Ausführungsformen der Erfindung anhand der Figuren 1 bis 4 im Detail erläutert.

Figur 1 zeigt schematisch den Aufbau einer vorteilhaften Ausführungsform der erfindungsgemäßen Vorrichtung.

Figur 2 zeigt einen vertikalen Längsschnitt durch eine Gießform einer vorteilhaften Ausführungsform der erfindungsgemäßen Vorrichtung.

Figur 3 zeigt einen horizontalen Längsschnitt durch eine Gießform einer vorteilhaften Ausführungsform der erfindungsgemäßen Vorrichtung.

Figur 4 zeigt ein Messergebnis in Form eines Graphen, bei dem die gemessene Zugkraft und die Temperatur über die Zeit für zwei verschiedene Versuche abgetragen sind.

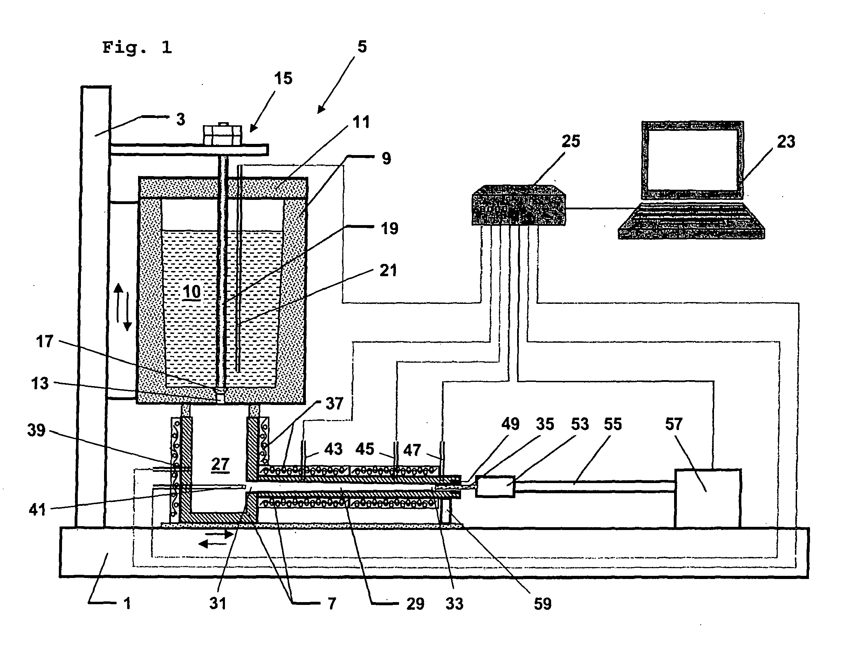

Figur 1 zeigt schematisch den gesamten Aufbau einer Vorrichtung zur Ermittlung der Heißrissempfindlichkeit von metallischen Schmelzen. Die wesentlichen Komponenten der Vorrichtung sind auf einer stabilen horizontalen Plattform 1 angeordnet. Von der horizontalen Plattform 1 erstreckt sich seitlich vertikal eine Halteeinrichtung 3, die dazu dient, ein Angusssystem 5 oberhalb einer Gießform 7 zu befestigen. Das Angusssystem 5 weist im Wesentlichen ein zylinderförmiges Behältnis auf, das als Schmelzofen 9 mit einer heizbaren Wandung ausgestaltet ist. Der Schmelzofen 9 hat eine obere Öffnung, durch die metallisches Material 10 eingefüllt werden kann, das innerhalb des Schmelzofens 9 geschmolzen werden kann. Die obere Einlassöffnung kann nach dem Befüllen mit einem Deckel 11 verschlossen werden. Unterseitig weist der Schmelzofen 9 eine Auslassöffnung 13 auf, die mit einem Verschlusssystem 15 geöffnet und geschlossen werden kann. Das Verschlusssystem 15 ist in dieser Ausführungsform ein Stopfen 17, der über einen vertikal verlaufenden Stab 19, der oberseitig durch eine zentrale Durchführung im Deckel 11 nach außen aus dem Schmelzofen 9 heraus geführt ist, gehoben und gesenkt werden kann. In der in Figur 1 gezeigten gesenkten Stellung des Stopfens 17 ist die unterseitige Auslassöffnung 13 verschlossen, und es kann keine metallische Schmelze 10 austreten. Zur Regelung der Heiztemperatur und/oder zur Bestimmung, wann die metallische Schmelze 10 die geeignete Temperatur erreicht hat, ist ein Temperaturmesselement 21 durch den Deckel 11 von oben endoskosisch oder teleskopisch in die metallische Schmelze 10 eingetaucht. Das Temperaturmesselement 21 ist dazu mit einem von einem Computer 23 gesteuerten Auslesesystem 25 über eine Kommunikationsverbindung verbunden.

Unterhalb der Auslassöffnung 13 des Schmelzofens 9 ist die Gießform 7 angeordnet, sodass bei Anhebung des Stopfens 17 die Schmelze 10 durch die Auslassöffnung 13 in die Gießform 7 fließen kann. Die Gießform 7 weist zwei Teilvolumen auf, wobei das erste Teilvolumen 27 im Wesentlichen ein zylindrisches Behältnis mit einer oberseitigen Öffnung zum Empfangen der Schmelze 10 aus dem Schmelzofen 9 ist. Ein zweites Teilvolumen 27 erstreckt sich in Form eines rohrförmigen Abschnitts 29 im Wesentlichen horizontal vom ersten Teilvolumen 27 weg. Der rohrförmige Abschnitt 29 hat ein erstes Ende 31, das mit einem unteren Bereich des ersten Teilvolumens 27 verbunden ist und ein zweites Ende 33, in das sich ein Messelement 35 erstreckt. Bei Befüllen der Gießform 7 aus dem oberseitigen Schmelzofen 9 füllt sich zunächst das erste Teilvolumen 27 bis eine Füllstandshöhe erreicht wird, bei dem die Schmelze 10 in den rohrförmigen Abschnitts 29 fließt. Für eine Messung muss der rohrförmige Abschnitt 29 vollständig gefüllt sein.

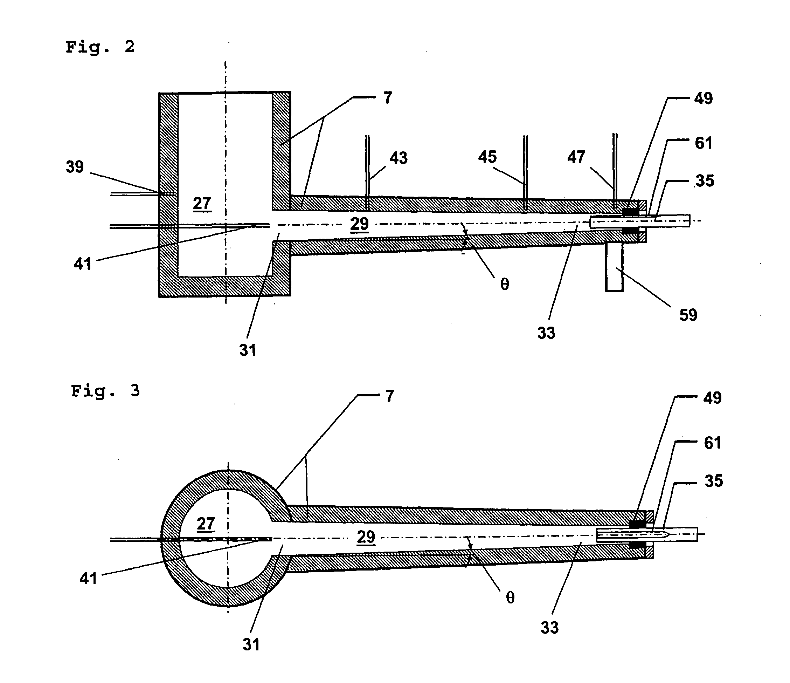

In den Figuren 2 und 3 noch deutlicher sichtbar, verringert sich die Querschnittsfläche des rohrförmigen Abschnitts 29 in jedem im Wesentlichen über die gesamte Länge beliebig auswählbaren Teilabschnitt des rohrförmigen Abschnitts 29 in Richtung des zweiten Endes 33 hin. In dieser Ausführungsfrom verringert sich der Durchmesser der kreisförmigen Querschnittsfläche über die gesamte Länge des rohrförmigen Abschnitts 29 mit einem Verjüngungswinkel von etwa 3° vom ersten Ende 31 zum zweiten Ende 33 hin.

Ein Großteil der Außenfläche der Gießform 7 ist mit einem Heiz- bzw. Kühlsystem 37 ausgestattet, sodass die Gießform 7 sowohl im ersten Teilvolumen 27 als auch im rohrförmigen Abschnitt 29 geeignet temperiert werden kann. Damit lässt sich gezielt das Erstarrungsverhalten der Schmelze 10 in der Gießform 7 steuern. Für die Regelung des Heiz- bzw. Kühlsystem 37 und zu Auswertungszwecken ist die Gießform 7 weiterhin mit Temperaturmesselementen 39, 41, 43, 45, 47 ausgestattet, die jeweils mit dem computergesteuerten Auslesesystem 25 über eine Kommunikationsverbindung verbunden sind. Ein Temperaturmesselement 39 misst die Temperatur des ersten Teilvolumens 27 der Gießform 7. Drei Temperaturmesselemente 43, 45, 47 messen die Temperatur des rohrförmigen Abschnitts 29 in verschiedenen Bereichen entlang der Erstreckungsrichtung des rohrförmigen Abschnitts 29. Ein Temperaturmesselement 47 ist dabei am zweiten Ende 33 des rohrförmigen Abschnitts 29 angeordnet. Ein Temperaturmesselement 41 ist durch eine Bohrung in der Gießform 7 teleskopisch oder endoskopisch durch das ersten Teilvolumen 27 in den Verbindungsbereich zwischen ersten Teilvolumen 27 und rohrförmigen Abschnitt 29 geführt. Damit lässt sich mit dem Temperaturmesselement 41 die Temperatur der Schmelze 10 am Hotspot messen.

Am zweiten Ende 33 des rohrförmigen Abschnitts 29 ist das Messelement 35, der sich im Wesentlichen in Form eines Kolbens koaxial in Erstreckungsrichtung des rohrförmigen Abschnitts 29 erstreckt und relativ zur Gießform 7 in einer Hülse 49 mit geringem Reibungskontakt, beispielsweise aus Graphit oder Keramik, geführt ist. Das Messelement 35 ist also in axialer Richtung beweglich gelagert. Zur Auslese der Kraft und/oder der Position in Erstreckungsrichtung des rohrförmigen Abschnitts 29 ist das Messelement 35 mit einer Messeinrichtung 51 gekoppelt. Die Messeinrichtung 51 weist dabei ein Universalgelenk 53 und ein stabförmiges Übertragungselement 55 auf, wobei das Universalgelenk 53 das Messelement 35 mit dem Übertragungselement 55 verbindet. Das Übertragungselement 55 überträgt die Kraft und/oder der Positionsänderung in axialer Richtung zu einem Kraft- bzw. Positionsmessgerät 57. Das Kraft- bzw. Positionsmessgerät 57 ist ebenfalls für die Auslese und Auswertung der Messdaten mit dem computergesteuerten Auslesesystem 25 über eine Kommunikationsverbindung verbunden.

Die Gießform 7 und das Kraft- bzw. Positionsmessgerät 57 haben definierte Positionen bezüglich der Plattform 1, mit der die vorzugsweise fest verbunden sind. Die Gießform 7 und/oder das Kraft- bzw. Positionsmessgerät 57 können auch in Erstreckungsrichtung des rohrförmigen Abschnitts 29 beweglich mit der Plattform 1 verbunden sein. Dann bedarf es zwar einer genauen Kalibration der axialen Positionen. Allerdings kann beispielsweise mit einem gesteuerten Schrittmotor eine zusätzliche Reißkraft auf die Schmelze 19 ausgeübt werden, um einen Riss zu provozieren und/oder die Belastbarkeit des Materials bei bestimmten Spannungen zu messen. Ebenso kann das Angusssystem 5 vertikal verstellbar mit der Halteeinrichtung 3 verbunden sein, damit die Fallhöhe der Schmelze 10 beim Anguss eingestellt und dem verwendeten Material entsprechend angepasst werden kann. Die gesamte Vorrichtung kann beispielsweise als ein Tischexperiment mit einem seitlichen Ausmaß von weniger als einem Meter ausgestaltet sein. Beispielsweise kann der sich zum zweiten Ende 33 hin verjüngende rohrförmige Abschnitt 29 eine Länge von etwa 20 cm und einen Durchmesser zwischen 12 und 8 mm haben.

Figuren 2 und 3 zeigen einen vertikalen bzw. horizontalen Längsschnitt durch die Gießform 7. Der Verjüngungswinkel θ des rohrförmigen Abschnitts 29 vom ersten Ende 31 zum zweiten Ende 33 hin ist zur Verdeutlichung dargestellt. Das zweite Ende 33 des im Wesentlichen horizontal verlaufenden rohrförmigen Abschnitts 29 ist von unten mit einer Abstützung 59 unterstützt, die mit der Plattform 1 verbunden ist, mit der auch das erste Teilvolumen 27 der Gießform 7 verbunden ist. Das Messelement 35 weist oberseitig eine in Erstreckungsrichtung des Messelements 35 verlaufende Lüftungsöffnung 61 in Form einer Kerbe auf. Die Lüftungsöffnung 61 erlaubt die Entfüftung des im Wesentlichen horizontal verlaufenden rohrförmigen Abschnitts 29 beim Befüllen mit der Schmelze 10.

Mit der gezeigten Vorrichtung wird nun das im Folgenden beschriebene Verfahren zur Ermittlung der Heißrissempfindlichkeit einer metallischen Schmelze ausgeführt. Zunächst wird ein zu prüfendes metallisches Material 10 mit bekannter chemischer Zusammensetzung, beispielsweise Magnesium mit einem Aluminiumanteil von 3%, in den Schmelzofen 9 gefüllt. Die Materialmenge 10 muss ausreichen, um in flüssiger Form die Gießform 7 ausreichend zu füllen, sodass der rohrförmige Abschnitts 29 vollständig gefüllt werden kann. Der Schmelzofen 9 wird dann auf eine Temperatur oberhalb des Schmelzpunktes des Materials 10 geheizt, beispielsweise auf 715°C. Gleichzeitig wird die Gießform 7 auf eine Temperatur von beispielsweise 350°C vorgeheizt, um das spätere Erstarrungsverhalten der Schmelze 10 in der Gießform 7 steuern zu können.

Sobald die Temperaturmesselemente 21, 39, 43, 45, 47 die geeignete Temperatur anzeigen, kann der Anguss beginnen. Über den Stab 19 wird der die unterseitige Auslassöffnung 13 des Schmelzofens 9 verschließende Stopfen 17 angehoben, sodass die metallische Schmelze 10 durch die Auslassöffnung 13 in das darunter befindliche erste Teilvolumen 27 der Gießform 7 fließen kann. Die Schmelze 10 verteilt sich dabei auch in den rohrförmigen Abschnitt 29, der mit seinem ersten Ende 31 mit dem ersten Teilvolumen 27 der Gießform 7 verbunden ist. Da die Temperatur der Gießform 7 unterhalb des Schmelzpunktes liegt, beginnt die Schmelze, in der Gießform 7 zu erstarren. Dies geschieht zunächst an der relativ zur Schmelze kälteren Wandung der Gießform 7 und vollzieht sich im Anschluss nach innen. Wegen des geringen Durchmessers am zweiten Ende 33 des rohrförmigen Abschnitts 29, des weitesten Abstands zum ersten Teilvolumen 27 und/oder wegen geeigneter Temperierung durch das Heiz- bzw. Kühlsystem 37 erstarrt die Schmelze 10 zunächst am zweiten Ende 33 des rohrförmigen Abschnitts 29, in das sich das Messelement 35 erstreckt. Durch die Erstarrung bildet sich eine kraftschlüssige und somit zugfeste Verbindung zwischen der erstarrten Schmelze 10 und dem Messelement 35. Beim Erstarren zieht sich das metallische Material 10 zusammen. Dies macht sich vor allem in der Erstreckungsrichtung des rohrförmigen Abschnitts 29 bemerkbar. Die Gießformm 7 selbst zieht sich ebenfalls unter der Abkühlung zusammen, allerdings um ein Vielfaches weniger als das Material 10, das einen Phasenübergang von flüssig nach fest durchläuft. Daher wird das Messelement 35 über die zugfeste Verbindung zwischen der erstarrten Schmelze 10 und dem Messelement 35 in den rohrförmigen Abschnitt 29 hineingezogen.

In radialer Richtung ist das Schrumpfen des erstarrenden Materials im rohrförmigen Abschnitt 29 kaum spürbar. Die Verjüngung des rohrförmigen Abschnitts 29 verhindert nun Reibungseffekte zwischen der Wandung der Gießform 7 und dem erstarrten Material 10. Durch den Verjüngungswinkel löst sich das Material nämlich durch das merklich Schrumpfen in axialer Richtung sofort von der Wandung und zieht sich nicht an dieser entlang. Die Messung der Zugkraft auf das Messelement 35 wird dadurch um ein Vielfaches genauer als bei der Verwendung von herkömmlichen Vorrichtungen. Es lässt sich nun über das Messelement 35 die Zugkraft messen, die die Schrumpfung des Materials 10 bewirkt oder die Längenänderung des Materials 10.

Während der Erstarrung wird auch die Temperatur am Hotspot, d.h. dem Verbindungsbereich zwischen ersten Teilvolumen 27 und rohrförmigen Abschnitt 29, aufgezeichnet. In diesem Bereich bleibt die Schmelze 10 am längsten flüssig und hier ist die Wahrscheinlichkeit für das Auftreten eines Heißrisses am größten. Für die Temperaturmessung wird ein Temperaturmesselement 41 durch eine Bohrung in der Gießform 7 teleskopisch oder endoskopisch durch das erste Teilvolumen 27 in den Verbindungsbereich zwischen ersten Teilvolumen 27 und rohrförmigen Abschnitt 29 geführt. Das Temperaturmesselement 41 ist hitzebeständig und mit entsprechendem Material wie z.B. Keramik ausgestattet.

Das von dem Computer 23 gesteuerte Auslesesystem 25 liest während der Erstarrung sowohl die mit den Temperaturmesselementen 39, 41, 43, 45, 47 gemessenen Temperaturen als Funktion der Zeit als auch die mit dem Kraft- bzw. Positionsmessgerät 57 gemessene Zugkraft auf das Messelement 35 oder dessen Positionsänderung.

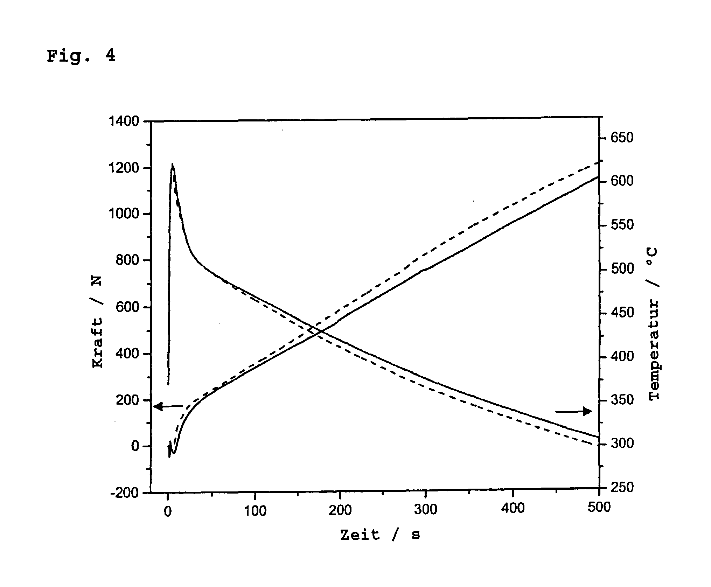

Figur 4 zeigt die Ergebnisse von zwei Versuchen, die jeweils mit einer Magnesiumschmelze mit einem Aluminiumanteil von 3%, einer Schmelztemperatur von 715 °C und einer Gießformtemperatur von 350°C durchgeführt wurden. Die Zugkraft und die Hotspot-Temperatur sind gegen die Zeit aufgetragen. Die durchgezogenen Kurven beziehen sich auf den ersten Versuch und die gestrichelten Kurven bezieht sich auf den zweiten Versuch. Die Pfeile zeigen jeweils auf die für die Kurven gültigen Achsen. Die Kraft nimmt nach einem steileren Anstieg zu Beginn der Erstarrungsphase in etwa kontant zu, wobei sich die Temperatur entsprechend entgegengesetzt verhält. Nach einem ersten Anstieg der Temperatur zu Beginn auf über 600 °C und einem steilen Abfall zu Beginn der Erstarrungsphase auf ca. 500 °C nimmt die Temperatur relativ konstant ab. Es zeigt sich, dass die erfindungsgemäße Vorrichtung erstmalig eine reproduzierbare quantitative Ermittlung der Heißrissempfindlichkeit erlaubt, da die Messergebnisse der beiden Versuche im Rahmen der Fehlermessung übereinstimmen.

Das Auftreten eines Heißrisses würde sich in der Kraftkurve als eine Stufe oder kurzen Abfall abzeichnen. Damit ist die maximale Zugkraft, die bei einem Versuch gemessen wird, davon abhängig, ob ein oder mehr Heißrisse aufgetreten sind und welche Form und Ausmaße diese haben. Damit ist die maximale Kraft, die nach einem definierten Erstarrungsintervall gemessen wird, ein quantitatives Maß für die Größe und/oder Anzahl von Heißrissen. Beispielsweise hat sich ergeben, dass bei einer Magnesiumschmelze mit einem Aluminiumanteil von 1% und einer Hotspot-Temperatur von 300°C eine Zugkraft von weniger als 900 N auf einen bestehenden großen Heißriss hindeutet. Optische Beobachtungen haben dies bestätigt. Ein nur mittelgroßer Riss erlaubt hierbei eine Zugkraft von 900 N bis 1100 N und ohne Heißriss oder mit nur sehr kleinen Heißrissen werden Zugkräfte von mehr als 1100 N gemessen.

## Patentansprüche

1. Vorrichtung zur Bestimmung der Heißrissempfindlichkeit von metallischen Schmelzen (10)
mit einer Gießform (7), die ein erstes Teilvolumen (27) und ein zweites Teilvolumen aufweist,
wobei das zweite Teilvolumen als ein rohrförmiger Abschnitt (29) mit einem ersten Ende (31) und einem zweiten Ende (33) ausgebildet ist,
wobei das erste Ende (31) mit dem ersten Teilvolumen (27) verbunden ist,
mit einem Messelement (35), das sich in das zweite Ende (33) des rohrförmigen Abschnitts (29) erstreckt und
mit einer Messeinrichtung (51) zum Erfassen einer Kraft auf das Messelement (35) und/oder einer Positionsänderung des Messelements (35) in Erstreckungsrichtung des rohrförmigen Abschnitts (29),
wobei die Messeinrichtung (51) mit dem Messelement (35) gekoppelt ist,
**dadurch gekennzeichnet,**
**dass** sich die Querschnittsfläche des rohrförmigen Abschnitts (29) in jedem im Wesentlichen über die gesamte Länge beliebig auswählbaren Teilabschnitt des rohrförmigen Abschnitts (29) in Richtung des zweiten Endes (33) hin verringert.

2. Vorrichtung nach Anspruch 1, **dadurch gekennzeichnet, dass** der rohrförmige Abschnitt (29) im Wesentlichen in horizontaler Richtung verläuft.

3. Vorrichtung nach Anspruch 1 oder 2, **dadurch gekennzeichnet, dass** Temperaturmesselemente (43, 45, 47) entlang der Erstreckungsrichtung des rohrförmigen Abschnitts (29) angeordnet sind.

4. Vorrichtung nach Anspruch 1 bis 3, **dadurch gekennzeichnet, dass** zumindest ein Temperaturmesselement (41) in dem ersten Teilvolumen (27) angeordnet ist.

5. Vorrichtung nach einem der Ansprüche 1 bis 4, **dadurch gekennzeichnet, dass** das erste Teilvolumen (27) mit einer Heizvorrichtung (37) versehen ist.

6. Vorrichtung nach einem der Ansprüche 1 bis 5, **dadurch gekennzeichnet, dass** das erste Teilvolumen mit einer Kühlvorrichtung (37) versehen ist.

7. Vorrichtung nach einem der Ansprüche 1 bis 6, **dadurch gekennzeichnet, dass** der rohrförmige Abschnitt (29) mit einer Heizvorrichtung (37) versehen ist.

8. Vorrichtung nach einem der Ansprüche 1 bis 7, **dadurch gekennzeichnet, dass** der rohrförmige Abschnitt (29) mit einer Kühlvorrichtung (37) versehen ist.

9. Vorrichtung nach einem der Ansprüche 1 bis 8, **dadurch gekennzeichnet, dass** am zweiten Ende (33) des rohrförmigen Abschnitts (29) eine Lüftungsöffnung (61) vorgesehen ist.
